# EUROPEAN PATENT SPECIFICATION

(11) **EP 1 946 117 B1**
(45) Date of publication and mention of the grant of the patent: **24.08.2011**
(21) Application number: 06837361.2
(22) Date of filing: 10.11.2006
(51) Int. Cl.: G01N 33/574, G01N 33/68

(54) **P66-SHC AS PREDICTIVE MARKER IN CANCER TREATMENT**
P66-SHC ALS PRÄDIKTIVER MARKER BEI DER KREBSBEHANDLUNG
P66-SHC EN TANT QUE MARQUEUR DE PRÉDICTION DANS LE TRAITEMENT DU CANCER

(30) Priority: 11.11.2005 US 735729 P; 12.09.2006 US 843788 P
(43) Date of publication of application: 23.07.2008
(73) Proprietor: Roger Williams Hospital, Providence, RI 02908-4735 (US)
(72) Inventor: FRACKELTON, A., Raymond, Jr., Rumford, Rhode Island 02916 (US)
(74) Representative: Lane, Cathal Michael
(86) International application number: PCT/US2006/043852
(87) International publication number: WO 2007/058965

(56) References cited:
- US-A1- 2004 033 542
- DAVOL P A ET AL: "SHC PROTEINS ARE STRONG, INDEPENDENT PROGNOSTIC MARKERS FOR BOTH NODE-NEGATIVE AND NODE-POSITIVE PRIMARY BREAST CANCER" CANCER RESEARCH, AMERICAN ASSOCIATION FOR CANCER RESEARCH, BALTIMORE, MD, US, vol. 63, no. 20, 15 October 2003 (2003-10-15), pages 6772-6783, XP008075232 ISSN: 0008-5472
- WANG HWEI-CHUNG ET AL: "Future prospects of neoadjuvant chemotherapy in treatment of primary breast cancer" SEMINARS IN SURGICAL ONCOLOGY, vol. 12, no. 1, 1996, pages 59-66, XP008075742 ISSN: 8756-0437
- HAYES D F ET AL: "Prognostic factors in breast cancer: current and new predictors of metastasis." JOURNAL OF MAMMARY GLAND BIOLOGY AND NEOPLASIA OCT 2001, vol. 6, no. 4, October 2001 (2001-10), pages 375-392, XP008075721 ISSN: 1083-3021
- DAVOL PAMELA A ET AL: "SHC ADAPTOR PROTEINS IN BREAST CANCER PROGNOSIS: NOVEL MOLECULAR MARKERS THAT PREDICT AGGRESSIVE STAGE 1 TUMORS" PROCEEDINGS OF THE ANNUAL MEETING OF THE AMERICAN ASSOCIATION FOR CANCER RESEARCH, NEW YORK, NY, US, vol. 43, March 2002 (2002-03), pages 44-ABSTRACT22, XP008075324 ISSN: 0197-016X
- FRACKELTON A RAYMOND JR ET AL: "p66 Shc and tyrosine-phosphorylated Shc in primary breast tumors identify patients likely to relapse despite tamoxifen therapy." BREAST CANCER RESEARCH : BCR 2006, vol. 8, no. 6, 2006, page R73, XP008075733 ISSN: 1465-542X
- 'Control', [Online] Dictionary Retrieved from the Internet: <URL:HTTP://WWW.BIOLOGY-ONLINE.ORG/DICTIONA RY/CONTROL> [retrieved on 2010-11-25]
- 'Control', [Online] Retrieved from the Internet: <URL:http/oxforddictionaries.com/view/entry /m_en_gb0176220m_en_gb0176220>
- 'Control', [Online] Retrieved from the Internet: <URL:http://www.collinslanguage.com/results .aspx?context=3&reversed=false&action=defin e&homonym=-1&text=control>

## Description

### BACKGROUND OF THE INVENTION

Currently available clinical and molecular markers do not adequately identify individuals with cancer who will benefit from particular therapies. Thus, a need exists for methods which can be used to predict whether an individual will benefit from a cancer treatment.

Davol PA et al, "She proteins are strong, independent prognostic markers for both node-negative and node positive primary breast cancer" [Cancer Res, v63, no 20, pp6772-6783] suggests that aggressive primary breast tumours are destined to recur if they display relatively high levels of PY-Shc relative to levels of p66 Shc.

### SUMMARY OF THE INVENTION

The invention in its broadest form is defined in independent claim 1:

An *in vitro* method for predicting whether an individual's chances of relapsing or dying from breast cancer are reduced two fold or more, compared to the general population of cancer patients having the same cancer or a substantially similar cancer, if the individual receives a treatment for breast cancer wherein the treatment comprises administering one or more agents to the individual, comprising:
determining the amount of p66-Shc present in cancerous cells of the individual,
wherein if the amount of p66-Shc present in the cancerous cells of the individual is lower than the amount of p66-Shc in a control sample taken from one or more individuals that do not have breast cancer, then the individual's chances of relapsing or dying from breast cancer are reduced if the individual receives the treatment.

Preferred embodiments are defined in dependent claims 2-12.

In particular embodiments, the cancer treatment comprises an adjuvant theraepy (e.g., adjuvant tamoxifen therapy; adjuvant chemotherapy). The amount of p66Shc can be determined using, for example, immunohistochemistry methods. For example, the amount of p66 She can be detected using an antibody or antigen binding fragment thereof that specifically binds to the p66Shc (e.g., a labeled monoclonal antibody).

### BRIEF DESCRIPTION OF THE DRAWINGS

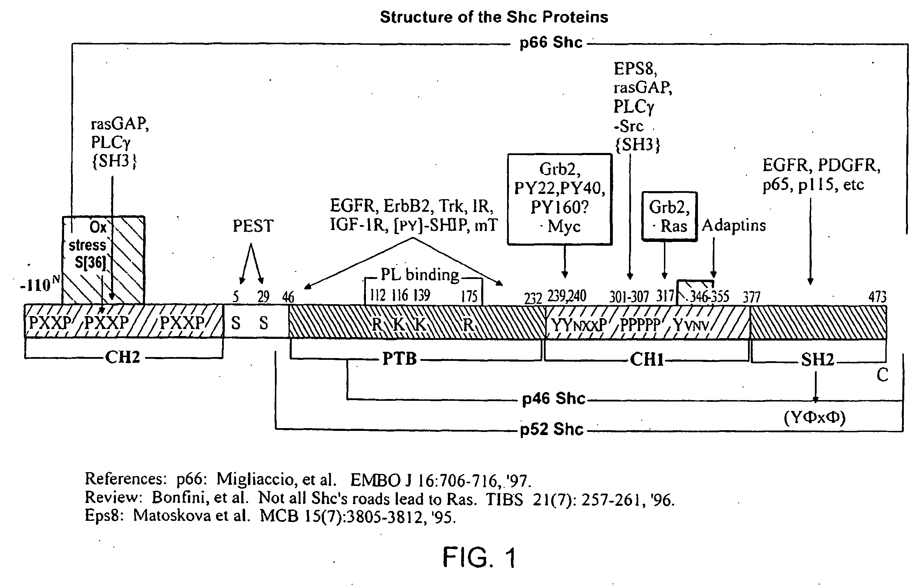

Fig. 1 is a schematic of the structure of the She proteins.

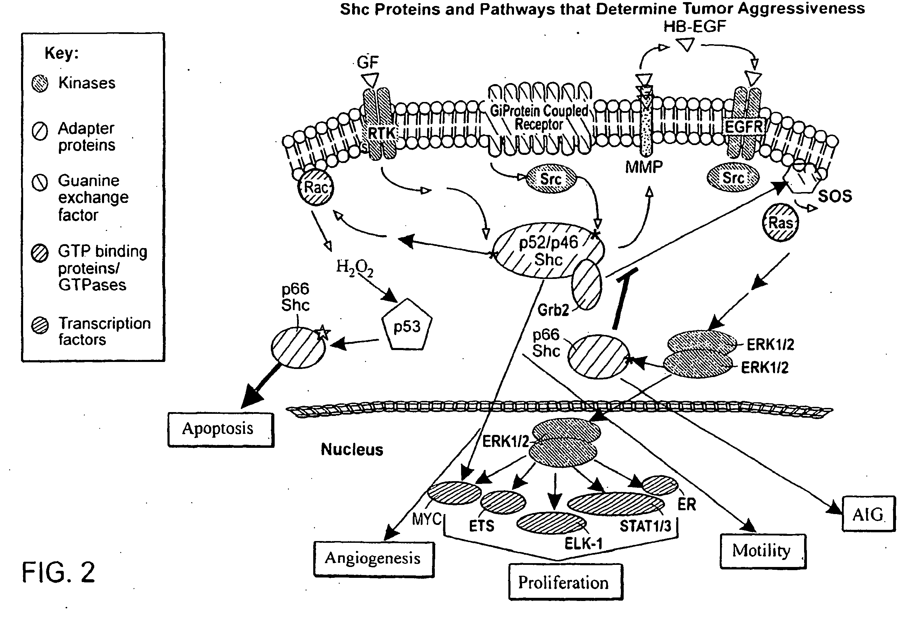

Fig. 2 is a schematic of She proteins and pathways that determine tumor aggressiveness.

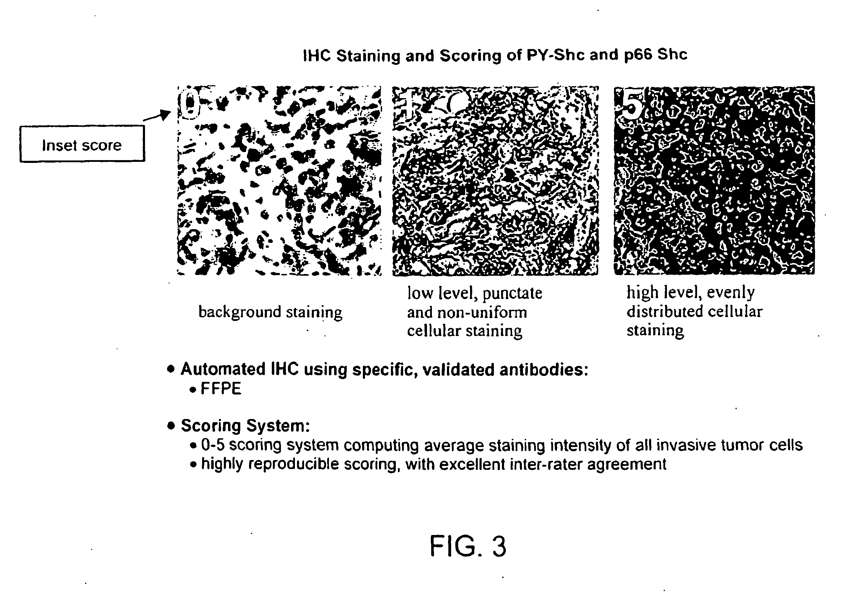

Fig. 3 shows immunohistochemistry staining and scoring of PY-She and p66 SHc.

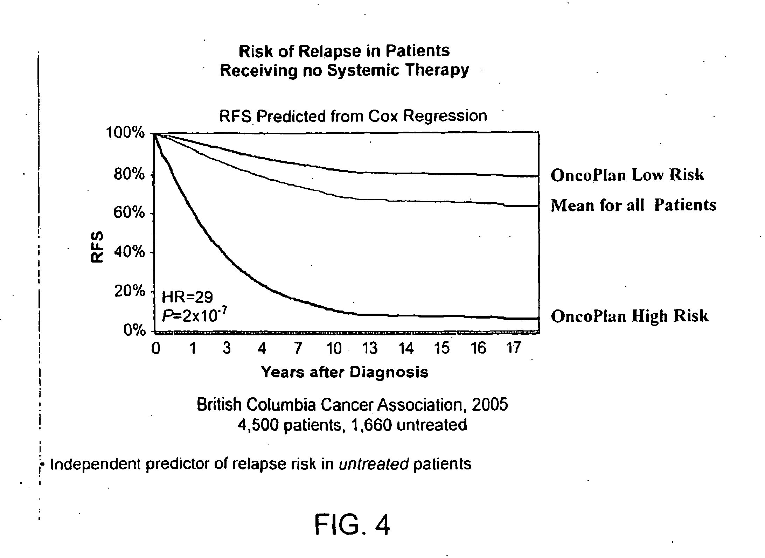

Fig. 4 is a graph of years after diagnosis versus relapse-free survival (RFS) in patients receiving no systemic therapy. Risk of relapse is shown for the range of risk predicted by the Shc proteins combined with independent covariates, nodal status and Her2 status.

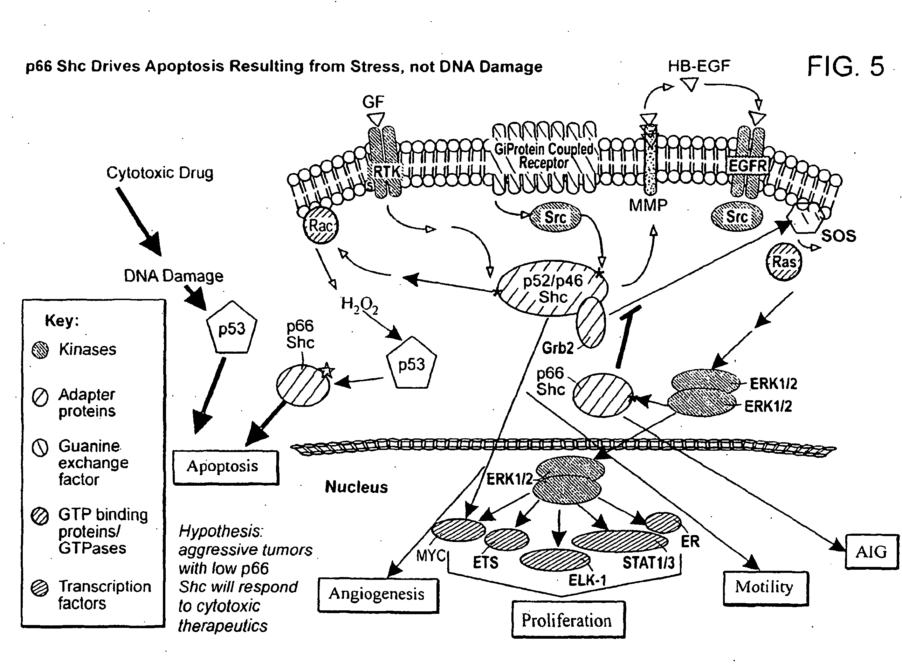

Fig. 5 is a schematic showing p66 Shc drives apoptosis resulting from stress, not DNA damage.

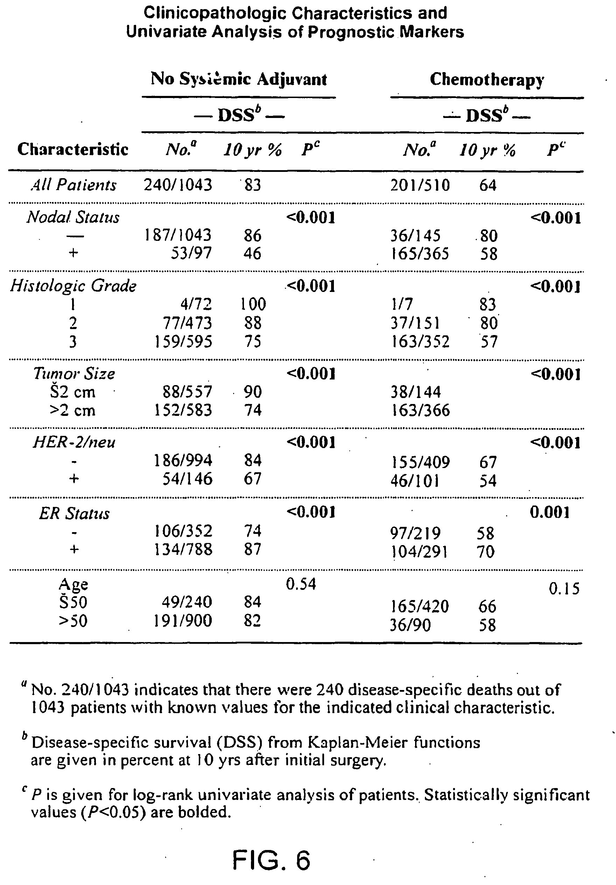

Fig. 6 shows clinicopathologic characteristics and univariate analysis of prognostic markers in patients who either received or did not receive adjuvant chemotherapy.

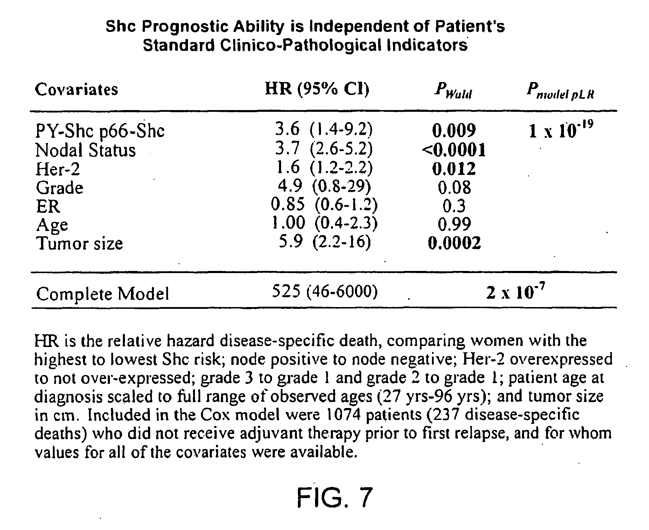

Fig. 7 shows that Shc prognostic ability is independent of patient standard clinico-pathological indicators.

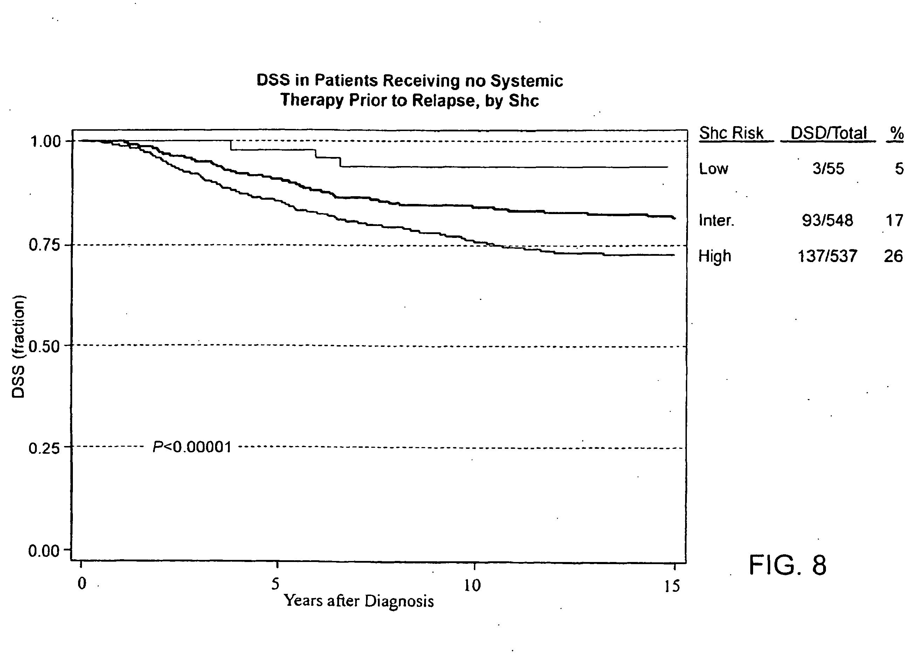

Fig. 8 is a graph of years in diagnosis versus disease specific survival (DSS) showing DSS in patients receiving no systemic therapy prior to relapse by Shc.

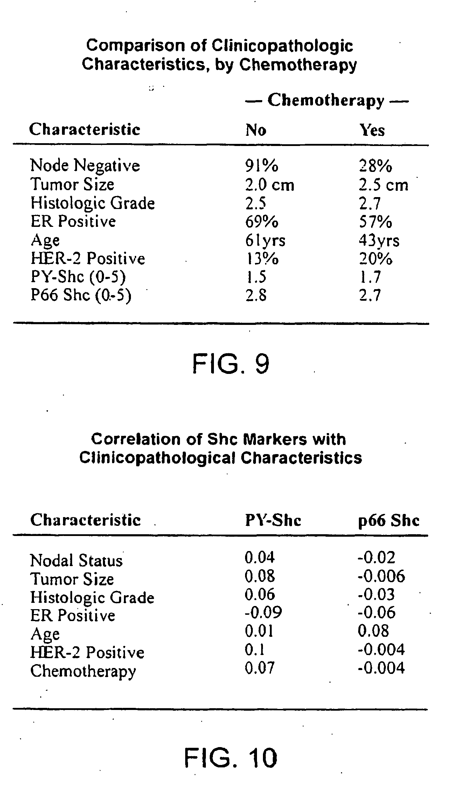

Fig. 9 shows comparison of clinicopathologic characteristics by chemotherapy.
Fig. 10 shows lack of correlation of Shc markers with clinicopathological characteristics.
Fig. 11 shows that p66 Shc interacts significantly with chemotherapy.
Fig. 12 are graphs showing that low expression of p66 Shc predicts benefit from chemotherapy. Shown are predicted hazard functions and risks of relapse for breast cancer patients determined from Cox proportional hazards models in which chemotherapy (±) was fully adjusted for tumor size (continuous variable), tumor grade, estrogen receptor (ER) status (binomial), Her2 status (binomial) and patient age at diagnosis. The high and low Shc risk categories correspond respectively to the lowest 60% of patients p66 Shc scores, and the highest 40% of patients p66 Shc scores.
Fig. 13 shows that patients whose tumors have low p66 Shc benefit most from cytotoxic therapy. Shown are the hazard ratio (HR) and P values corresponding to the relapse-free survival (RFS) functions shown in Fig. 13. The HRs and P values were obtained from Cox proportional hazards models in which chemotherapy was fully adjusted for all of the above mentioned covariates for all patients considered together (ALL) or in the low or high p66 Shc subgroups.
Fig. 14 is a bar graph showing that OncoPlan^{™} identifies node positive patients who will benefit most from chemotherapy.
Fig. 15 is another bar graph showing low expression of p66 She predicts benefit from chemotherapy.
Fig. 16 are graphs showing relapse-free survival (RFS) and hazard functions by tamoxifen therapy by p66 She levels (in quartiles). The hazard and relapse free survival (RFS) were predicted from Cox proportional hazards models in which tamoxifen (±) was fully adjusted for tumor size (as a continuous variable), tumor grade, estrogen receptor (ER) status (as a binomial), Her2 status (as a bionomial) and patient age at diagnosis.

### DETAILED DESCRIPTION OF THE INVENTION

Shc proteins are cellular molecules implicated in many pathways associated with cancers. Numerous growth factors and their receptors have been implicated in breast cancer development and aggressiveness, including Her-2/neu, other members of the EGF3-receptor family, IGF-1, and many others (Dickson, R. B. and Lippman, M. E., Endocr- Rev, 16: 559-589 (1995); Tuck, A. B. et al., Am J Pathol, 148:225-232 (1996); Nolan, M. K., et al., Int J Cancer, 72: 828-834 (1997); Mitchell, P. J. et al., Oncogene, 9: 2383-2390 (1994); Luttrell, D. K. et al., Proc Natl Acad Sci, USA, 91: 83-87 (1994)). Shc is a second messenger adapter protein, which becomes tyrosine phosphorylated in response to these receptors, from many G-protein-coupled receptors, and in response to cellular interactions with the extracellular matrix (Pelicci, G., et al., Cell, 70: 93-104 (1992); Filardo, E. J. et al., Mol Endocrinol, 14:1649-1660 (2000); Ravichandran, K. S., Oncogene, 20: 6322-6330 (2001); Luttrell, L. M., et al., Adv Second Messenger Phosphoprotein Res, 31:263-277 (1997)). Shc is involved also in responses to stimuli that activate cell proliferation, invasion, motility and control anchorage independent growth (Buday, L., et al., Oncogene, 11: 1327-1331 (1995); Dong, C., et al., JBiol Chem, 271:6328-6332 (1996); Hashimoto, A., et al. J Biol Chem, 274:20139-20143 (1999); Gotoh, N., et al. Embo J, 15:6197-6204 (1996); Gotoh, N., et al., Mol Cell Biol, 17:1824-1831 (1997); Panchamoorthy, G., et al., JBiol Chem, 271:3187-3194 (1996); Baumann, C. A., et al., Nature, 407:202-207 (2000); Matoskova, B., et al., Mol Cell Biol, 15:3805-3812 (1995); Scita, G., et al., Nature, 401:290-293 (1999); Gu, H., et al., Mol Cell Biol, 20:7109-7120 (2000); Nguyen, D. H., et al., JBiol Chem, 275:19382-19388 (2000); Gu, J., et al., J Cell Biol, 146:389-403 (1999); Songyang, Z., et al., Cell, 72:767-778 (1993)). Although tyrosine phosphorylation of the 52- and 46-kDa isoforms of Shc (PY-Shc) appears to drive these reactions forward, the alternative, 66-kDa Shc isoform (p66 Shc) appears to inhibit some of these processes (Migliaccio, E., et al., Embo J, 16:706-716 (1997); Okada, S., et al., J Biol Chem, 272:28042-28049 (1997)); additionally, p66 Shc is an apoptotic sensitizer to oxidative stress (Migliaccio, E., et al., Nature, 402:309-313 (1999); Nemoto, S. and Finkel, T., Science, 295:2450-2452 (2002)), which may result from chronic activation of growth-factor pathways, by infiltrating neutrophils and macrophages, and by neo-vascularization of hypoxic tumors (Brown, N. S. and Bicknell, R., Breast Cancer Res, 3:323-327 (2001); Irani, K.et al., Science, 275:1649-1652 (1997)). It has been previously shown that the levels of PY-Shc and p66 Shc expressed in a patient's tumor cells would serve as a marker for disease aggressiveness (U.S. Published Application No. 2004/0033542 A1 .

Shown herein is that p66 proteins can be used to predict whether an individual will be likely to benefit from a treatment for cancer. The invention has been demonstrated herein using studies in which tumors from women who were diagnosed with invasive breast cancer, some of whom received chemotherapy as part of their initial treatment, were examined for the presence of p66 Shc and PY-Shc. Patients who had low levels of p66 Shc and did not receive chemotherapy had very poor outcomes. However, the chances of similar patients who received chemotherapy relapsing and dying from their disease were reduced by two-fold or more. Conversely, women with high levels of p66 Shc had a much higher likelihood of surviving their disease, but appeared to derive no benefit from chemotherapy.

Accordingly, the present invention provides a method for predicting whether an individual (*e.g*., patient) will benefit from a (one or more) treatment for cancer comprising determining the amount of p66-Shc present in cells of the individual. In a particular embodiment, the methods comprise determining the amount of p66-Shc present in cancerous cells of the individual. If the amount of p66-Shc present in the cells of the individual is lower than the amount of p66-Shc in a control, then the individual will likely benefit from the treatment (conversely, if the amount of p66-Shc present in the cells of the individual is higher than the amount of p66-Shc in a control, then the individual will likely not benefit from the treatment).

In particular, the methods described herein can be used to predict the cancer patients who are likely (or not likely) to respond to, or derive a benefit from, a cancer therapy based on the individual's level of p66 Shc. That is, as shown herein, cancer patients that have low levels of p66 Shc compared to a suitable control indicates that the cancer patient has a 2-fold or greater (*e.g*., 3-fold, 4-fold, 5-fold) chance of benefiting from (having a favorable response to) a particular cancer treatment compared to the general population of cancer patients (in particular, cancer patients having the same cancer or a substantially similar cancer). As also shown herein, cancer patients that have low levels of p66 Shc compared to a suitable control indicates that the cancer patient has from about a 50% to about a 75% chance of benefiting from (having a favorable response to) a particular cancer treatment compared to a control (*e.g*., the general population of cancer patients, and in particular embodiments, the general population of cancer patients having the same cancer or a substantially similar cancer). In other embodiments, cancer patients that have low levels of p66 SHc compared to a control have from about a 55% to about a 70% chance, or from about a 60% to about a 65% chance, of benefiting from a particular cancer treatment compared to a control.

Thus, the methods described herein can be used to identify a cancer patient as a suitable candidate for a cancer treatment comprising determining the amount of p66-Shc present in cells of the individual. In a particular embodiment, the methods comprise determining the amount of p66-Shc present in cancerous cells of the individual. If the amount of p66-Shc present in the cells of the individual is lower than the amount of p66-Shc in a control, then the cancer patient is a suitable candidate for a cancer treatment.

Alternatively, the methods described herein can be used to identify or classify a cancer patient as a member of a class of cancer patients that will likely benefit from a cancer treatment comprising determining the amount of p66-Shc present in cells of the cancer patient. In a particular embodiment, the methods comprise determining the amount of p66-Shc present in cancerous cells of the cancer patient. If the amount of p66-Shc present in the cells of the cancer patient is lower than the amount of p66-Shc in a control, then the cancer patient will benefit from the cancer treatment.

In particular embodiments, the methods described herein can be used to identify cancer patients that would be suitable for enrollment in (and cancer patients who are not suitable for enrollment in) clinical trials being conducted to assess or identify new cancer therapies.

The methods described herein can also be used to identify a (one or more) tumor or a tumor cell that can likely be successfully targeted by an anti-cancer agent, such as a chemotherapeutic agent, comprising determining the amount of p66-Shc present in a cell of the tumor or in the tumor cell. If the amount of p66-Shc present in the cell of the tumor or the tumor cell is lower than the amount of p66-Shc in a control, then the tumor or tumor cell can be successfully targeted by the anti-cancer agent. For example, the methods described herein can be used to identify a tumor or a tumor cell which will respond to the anti-cancer effect(s) of a chemotherapeutic agent (*e.g*., the damaging effects of chemotherapeutic agents on dividing cells, such as cancer cells). Such anti-cancer effects include interaction with a cancer cell's DNA, RNA and/or mitotic machinery to prevent the cancer cell from reproducing, interfering with an enzyme involved in a cancer cell's DNA repair mechanism, interfering with an enzyme involved in a cancer cell's replication, and causing death of a cancer cell (apoptosis).

As described herein, a "cancer therapy", "treatment for cancer" or "cancer treatment" comprises administering one or more agents to the individual. In one embodiment, the agent is a chemotherapeutic agent which targets cancer cells that divide rapidly. Examples of such agents include an alkylating agent (*e.g*., busulfan, cisplatin, carboplatin, chlorambucil, cyclophosphamide, ifosfamide, dacarbazine (DTIC), mechlorethamine (nitrogen mustard), melphalan, temozolomide); a nitrosoureas (*e.g*., carmustine (BCNU), lomustine (CCNU)); an antimetabolite (*e.g*., 5-fluorouracil, capecitabine, 6-mercaptopurine, methotrexate, gemcitabine, cytarabine (ara-C), fludarabine, pemetrexed); an anthracycline or related drug (*e.g*., daunorubicin, doxorubicin (adriamycin), epirubicin, idarubicin, mitoxatrone); a topoisomerase (topoisomerase I inhibitor; topoisomerase inhibitor II) inhibitor (*e.g*., topotecan, irinotecan, etoposide (VP-16), teniposide); and a mitotic inhibitor (*e.g*., taxanes (paclitaxel, docetaxel, taxol) vinca alkaloids (vinblastine, vincristine, vinorelbine).

In another embodiment, the agent is a targeted therapy agent which attacks cancer cells more specifically than chemotherapeutic agents. Examples of such agents include imatinib (Gleevec), gefitinib (Iressa), erlotinib (Tarceva), rituximab (Rituxan), and bevacizumab (Avastin).

In yet another embodiment, the agent is a sex hormone, or hormone-like drug that alters the action or production of female of male hormones and can be used to slow the growth of breast, prostate, and endometrial (uterine) cancers. Examples of such agents include anti-estrogens (*e.g*., tamoxifen, fulvestrant), aromatase inhibitors (*e.g*., anastrozole, exemestane, letrozole), progestins (*e.g*., megestrol acetate), antiandrogens (*e.g*., bicalutamide, flutamide) and LHRH agonists (leuprolide, goserelin).

In addition, the agent can be a drug which is sued to stimulate the immune system to more effectively recognize and attack cancer cells of an individual with cancer.

Other examples of agents include a hormone such as a selective estrogen receptor modulator (SERM); an antibody or antigen binding fragment thereof (*e.g*., herceptin); a protein tyrosine kinase inhibitor; a combination of chemotherapeutic agents such as cytoxan (C), methotrexate (M), fluorouracil (F), an anthracylcin such as adriamycin (A), epirubicin (E), doxorubicin, and/or daunorubicin; a targeted kinase inhibitor; a metalloproteinase inhibitor; and a proteosome inhibitor.

In a particular embodiment, the present invention provides a method for predicting whether an individual (*e.g*., patient) will benefit from a (one or more) treatment for cancer, wherein the treatment comprises regional radiation (as opposed to local radiation used as a primary treatment for cancer).

The one or more agents can be administered to the individual before (*e.g*., neoadjuvant therapy), during or after (*e.g*., adjuvant therapy) primary treatment of the cancer. As used herein "primary treatment of a cancer" generally refers to a treatment involving surgery (*e.g*., surgical removal of a tumor) and/or radiation (*e.g*., local radiation). In the methods of the present invention, the "treatment for cancer" can be a neoadjuvant treatment wherein an agent (*e.g*., a hormone, a chemotherapeutic) is administered prior to surgical removal of a (residual) tumor, or prior to localized radiation (*e.g*., radiation used to shrink the tumor in order to simplify subsequent surgical removal of the tumor). In another embodiment, the "treatment for cancer" can be an adjuvant treatment wherein an agent (*e.g*., a hormone, a chemotherapeutic agent) is administered after surgical removal of a (residual) tumor, or after localized radiation. In yet another embodiment, the treatment can be a palliative treatment in which one or more chemotherapeutic agents are administered to an individual to treat metastatic cancer (e.g., to make the individual more comfortable and/or to prolong survival of the individual after the cancer has metastasized).

The invention in its broadest form is defined in independent claim 1:

An *in vitro* method for predicting whether an individual's chances of relapsing or dying from breast cancer are reduced two fold or more, compared to the general population of cancer patients having the same cancer or a substantially similar cancer, if the individual receives a treatment for breast cancer wherein the treatment comprises administering one or more agents to the individual, comprising:
determining the amount of p66-Shc present in cancerous cells of the individual,
wherein if the amount of p66-Shc present in the cancerous cells of the individual is lower than the amount of p66-Shc in a control sample taken from one or more individuals that do not have breast cancer, then the individual's chances of relapsing or dying from breast cancer are reduced if the individual receives the treatment.

Preferred embodiments are defined in dependent claims 2-12.

In one embodiment, the present invention is directed to a method for predicting whether an individual will benefit from a (one or more) chemotherapy treatment (*e.g*., adriamycin cytoxan (AC), cytoxan, methotrexate, fluorouracil (CMF)) for cancer comprising determining the amount of p66-Shc present in cancerous cells of the individual. If the amount of p66-Shc present in the cells of the individual is lower than the amount of p66-Shc in a control, then the individual will likely benefit from the chemotherapy treatment.

In a particular embodiment, the present invention is directed to a method for predicting whether an individual will benefit from adriamycin cytoxan (AC) for breast cancer comprising determining the amount of p66-Shc present in cancerous cells of the individual. If the amount of p66-Shc present in the cells of the individual is lower than the amount of p66-Shc in a control, then the individual will likely benefit from the AC treatment.

In another embodiment, the present invention is directed to a method for predicting whether an individual will benefit from a cytoxan, methotrexate, fluorouracil (CMF) treatment for breast cancer comprising determining the amount of p66-Shc present in cancerous cells of the individual. If the amount of p66-Shc present in the cells of the individual is lower than the amount of p66-Shc in a control, then the individual will likely benefit from the CMF treatment.

The present invention also encompasses a method for predicting whether an individual will benefit from hormonal (*e.g*., estrogen receptor targeted therapies such as tamoxifen) treatment for breast cancer comprising determining the amount of p66-Shc present in cancerous cells of the individual. If the amount of p66-Shc present in the cells of the individual is lower than the amount of p66-Shc in a control, then the individual will likely benefit from the tamoxifen treatment.

As used herein "p66-Shc" refers to a 66 kD isoform of the adaptor protein designated "Shc". As also used herein "phosphorylated Shc (PY-Shc)" refers to an adaptor protein designated "Shc" having at least one of its amino acid residue side chains phosphorylated. In a particular embodiment, a tyrosine residue of Shc is phosphorylated ("tyrosine phosphorylated Shc"). Such residues include, for example, tyrosine residue 317.

The amount of p66-Shc and/or phosphorylated-Shc present in a cell can refer to either an absolute amount as measured in molecules, moles or weight per unit volume or cell, or a relative amount as designated, for example, by a numerical rating from about 0 to about 5 obtained as the average intensity of an immunohistochemical stain that is specific for p66 Shc or PY-Shc. This intensity is determined relative to a standard (breast cancer tissue or calibrated tissue culture cells that express known amounts of p66 Shc and/or PY-Shc). Another similar method employs immunofluorescence detection and either subjective semiquantitative estimation of staining intensity, or automated measurement of staining intensity.

In the methods of the present invention, variables (*e.g*., risk factors) in addition to the amount of p66-Shc and/or phosphorylated-Shc present in a cell of an individual can be used to predict whether an individual will benefit from a treatment for cancer. Examples of additional variables which can be used include the individual's age, sex, overall health, family history, genotype, nodal status, tumor size, tumor grade, age, estrogen receptor (ER) status, marker expression (e.g., Her2 expression) and combinations thereof.

The methods described herein can be used to predict whether an individual that has been diagnosed with cancer (a cancer patient), is susceptible to cancer, and/or is at high risk for developing cancer (*e.g*., at risk of developing a hereditary form of cancer), will benefit from one or more cancer treatments. In addition, the methods described herein can be used to predict whether an individual will benefit from a treatment for cancer wherein the treatment comprises the administration of any single agent or combination of agents (*e.g*., chemotherapeutic drugs in which one of the drugs is an anthracycline (*e.g*., doxorubicin, epirubicin, daunorubicin) to the individual.

In the methods of the present invention, the individual can be a mammal such as a primate (*e.g*., human, chimpanzee, ape, monkey), a canine, a feline, a rodent, a porcine, an ovine, and a bovine.

The methods described herein can be performed on an individual or on a sample of an individual who has not been previously treated for cancer, that is, to identify an individual who will benefit from a primary treatment for cancer. For example, the methods described herein can be used to identify whether an individual having cancer (*e.g*., inflammatory breast cancer) will benefit from a primary chemotherapy treatment. Alternatively, the methods described herein can be performed on an individual or on a sample of an individual who has previously been treated for cancer, that is, to identify an individual who will benefit from an adjuvant treatment for cancer. For example, the methods described herein can be used to identify whether an individual having cancer will benefit from an adjuvant cancer therapy (chemotherapeutic; hormonal) after the patient has received a primary treatment for cancer such as surgery and/or radiation. Thus, the methods described herein can be performed as an early stage intervention for an early stage cancer diagnosis, as well as a later stage intervention for a more developed cancer, including metastatic cancer (*e.g*., AJC stages 1-4). In a particular embodiment, the individual has had surgery (the tumor has been removed surgically) and/or radiation to treat a tumor, and if the amount of p66-Shc is lower than the amount of p66-Shc in a control, and/or the amount of phosphorylated Shc is higher than the phosphorylated Shc present in a control in this individual, then the individual is deemed as having a high risk of recurrence of the cancer, and that individual will benefit from a cancer treatment.

As used herein, a "cancer" includes any type of cancer that is associated with abnormal levels of Shc proteins. In one embodiment, the methods described herein can be used to predict whether an individual will benefit from treatment for a cancer that is associated with abnormal levels of p66 Shc protein compared to a control. In another, the methods described herein can be used to predict whether an individual will benefit from treatment for a cancer that is generally treated with the agents described herein (*e.g*., tamoxifen, AC, CMF) either before, during or after primary treatment of the cancer. Examples of the types of cancers for which the methods described herein can be used include breast cancer (e.g., inflammatory breast cancer), ovarian cancer, prostate cancer, endometrial cancer, gastric cancer, bladder cancer, kidney cancer, pancreatic cancer, lung cancer, brain cancer, skin cancer (*e.g*., melanoma) and metastatic cancer. In one embodiment, the cancer manifests as a tumor, and the cancerous cells are tumor cells derived from the cancerous tumor. As used herein a "cancerous tumor cell" refers to a cancerous cell within, or originating from, a tumor. Cancerous tumor cells are distinct from other, non-cancerous cells present in a tumor, such as vascular cells. In another embodiment, the cancer is metastasized.

As will be apparent to one of skill in the art, an individual "will benefit from"; "will derive a benefit from"; "respond to"; "respond favorably to", or "have a favorable response (outcome) to" a cancer treatment when the cancerous condition is prevented, eliminated (*e.g*., in remission), diminished (*e.g*., a reduction in the size and or mass of the tumor), or under control (*e.g*., the cancer is contained, not spreading beyond the primary site or not further metastasizing) in the individual after treatment.

The amount of p66-Shc and/or phosphorylated-Shc can be determined using a variety of methods known in the art. For example, the determination step can comprise the use of flow cytometry or immunohistochemistry. In another embodiment, an antibody (*e.g*., a detectable antibody) that specifically binds to (has binding specificity for) p66-Shc or phosphorylated Shc can be used. As used herein an "antibody" includes, by way of example, both naturally occurring and non-naturally occurring antibodies. Specifically, this term includes polyclonal and monoclonal antibodies, and fragments thereof. Furthermore, this term includes chimeric antibodies and wholly synthetic antibodies, and fragments thereof (e.g., antigen-binding fragments). Additionally, this term includes anti-mimetopes isolated from random peptide libraries where such random peptides specifically bind to PY-Shc or to p66 Shc.

In a particular embodiment, the Shc Test^{™}, also referred to herein as the OncoPlan^{™} (Catalyst Oncology, Worcester, MA) is used to determine the amount of p66-Shc and/or phosphorylated-Shc (see *e.g*., U.S. Published Application No. 2004/0033542 A1 and U.S. Published Application No. 2005/004008 A1. OncoPlan^{™} measures the two forms of Shc protein described herein, drive the formation of protein complexes involved in signal transduction pathways and have been found to be involved in many of the pathways important to development of aggressive cancer. These two forms have a "push pull" relationship with each other: tyrosine-phosphorylated (PY)-Shc helps drive these dangerous cell pathways, but p66 Shc, after initial stimulation, works to inhibit the very growth pathway the other Shc proteins promote.

The method of determining the amount of p66-Shc and/or phosphorylated-Shc can be performed on any suitable sample (biological sample) obtained from the individual that comprises cancerous cells, and will likely depend on the cancer diagnosis. For example, the sample can be cells, tissues, blood, lymph, spinal fluid, semen, saliva, mucus, urine and feces. In a particular embodiment, the sample is a formalin fixed and/or paraffin-embedded tumor tissue from a biopsy or surgical resection of a cancer (*e.g*., tumor).

Any suitable control can be used wherein the amount of p66-Shc and/or phosphorylated-Shc in the control sample is indicative of the amount of p66-Shc and/or phosphorylated-Shc in an individual (one or more) that does not have cancer (*e.g*., the amount of p66-Shc and/or phosphorylated-Shc in one or more healthy individuals). For example, a suitable control can be established by assaying one or more (e.g., a large sample of) individuals which do not have cancer and using a statistical model to obtain a control value (standard value; known standard). See, for example, models described in Knapp, R.G. and Miller M.C. (1992) *Clinical*

*Epidemiology and Biostatistics,* William and Wilkins, Harual Publishing Co. Malvern, PA . Thus, as used herein, a "control" or "known standard" can refer to one or more of an amount, ratio or distribution, as applicable, with regard to p66-Shc and phosphorylated Shc. A control or known standard can reflect such amount, ratio and/or distribution characteristic of an individual that does not have cancer.

The invention is illustrated in the Examples section which follows. This section is set forth to aid in an understanding of the invention but is not intended to, and should not be construed to limit in any way the invention as set forth in the claims which follow thereafter.

### EXEMPLIFICATION

### Example 1

Shc Proteins in Primary Breast Tumors Predict Outcome in Treatment Naïve, Tamoxifen-Treated and Chemotherapy-Treated Patients

Background: Better predictors of benefit from adjuvant hormonal or chemotherapy are needed for breast cancer patients. The Shc signaling-adapter proteins, implicated in many pathways associated with aggressive cancers, can be measured in tumor specimens by the immunohistochemical Shc Test^{™} that reports activated, tyrosine phosphorylated (PY)-Shc, and an inhibitory form of this protein, p66 Shc. Previous studies indicated that the Shc Test has strong abilities to predict disease outcome. Described herein is the validity of its pure prognostic ability both in naïve patients and patients treated with an adjuvant therapy, and report its ability to identify those patients most likely to benefit from adjuvant tamoxifen or chemotherapies.

Materials and Methods: Patient samples included primary invasive tumor specimens from 4,326 British Columbian women in Tumor Micro-Array (TMA) format (no adjuvant therapy=1860; tamoxifen=1366; chemotherapy (AC, CMF, FAC, CEF) =744; relapsed=1752; died of disease=1334, average follow-up 13 yrs). Two additional sources were 65 primary tumor specimens from tamoxifen-only-treated patients (10 recurrences, 6.4 yrs average follow-up) from Roger Williams Medical Center (RWMC), and 32 (14 recurrences) from a published study (Massachusetts General Hospital (MGH)) (Ma et al., Cancer Cell, 5:1-10 (2004)). Immunohistochemistry (IHC) staining of the Shc proteins was scored on a continuous 0-5 scale by two pathologists, blinded to patient clinical data.

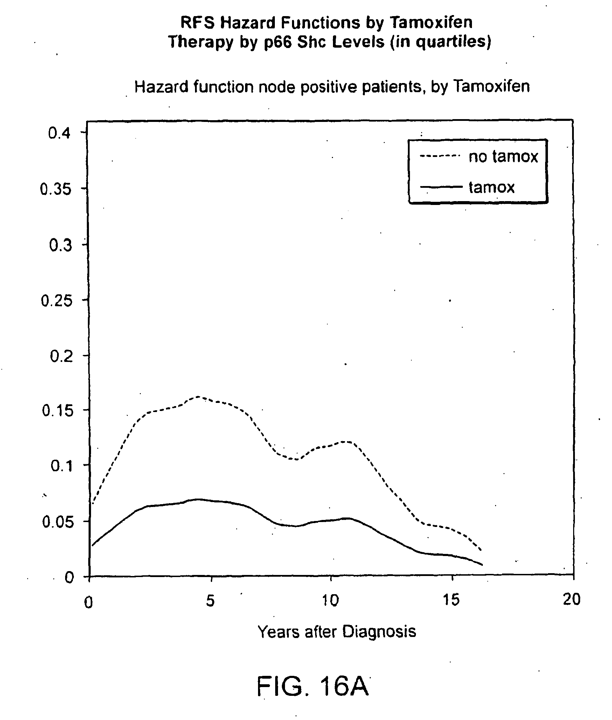

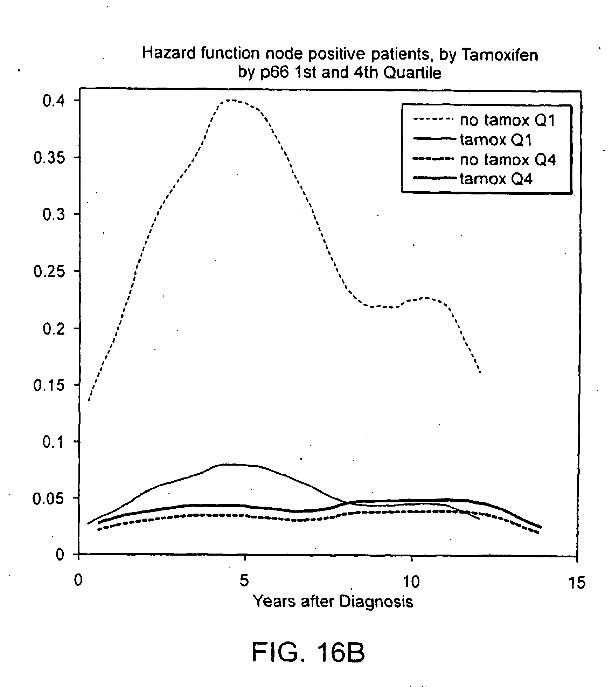

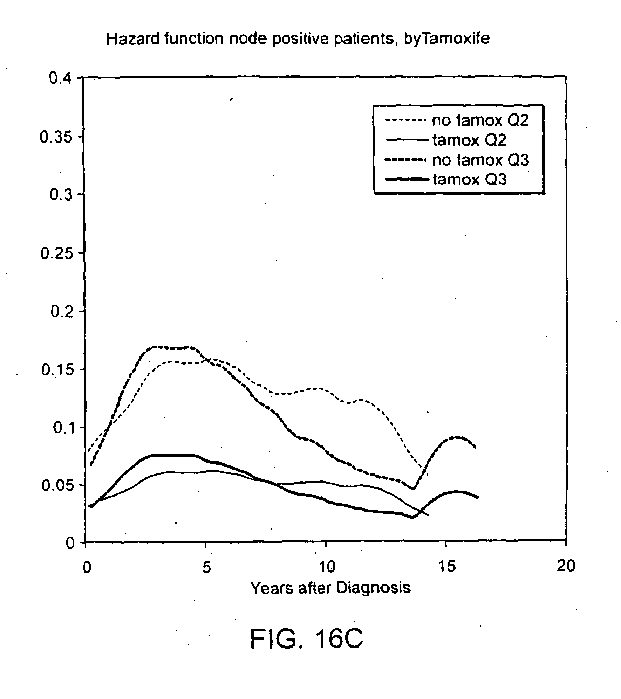

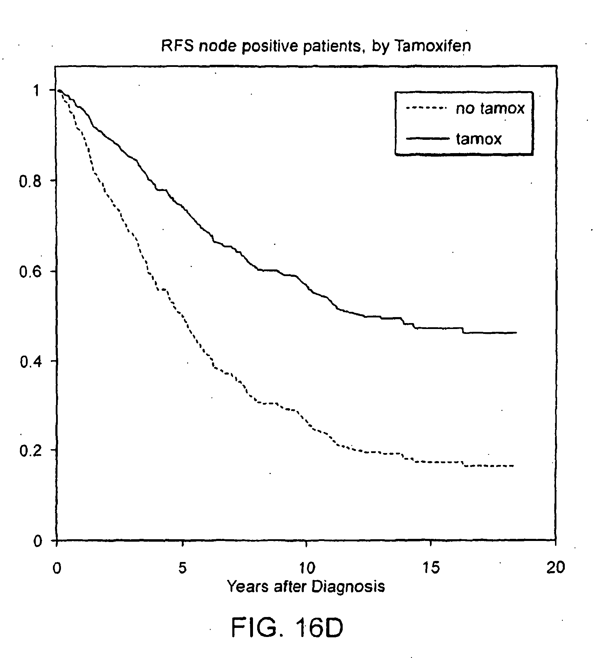

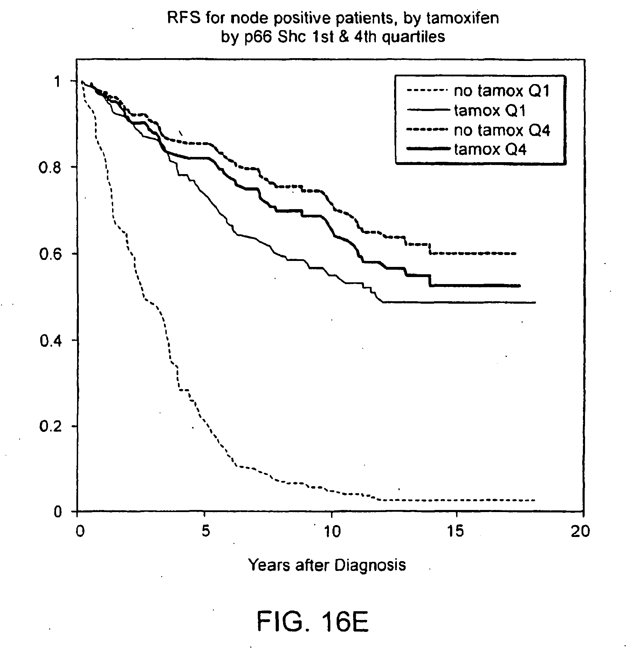

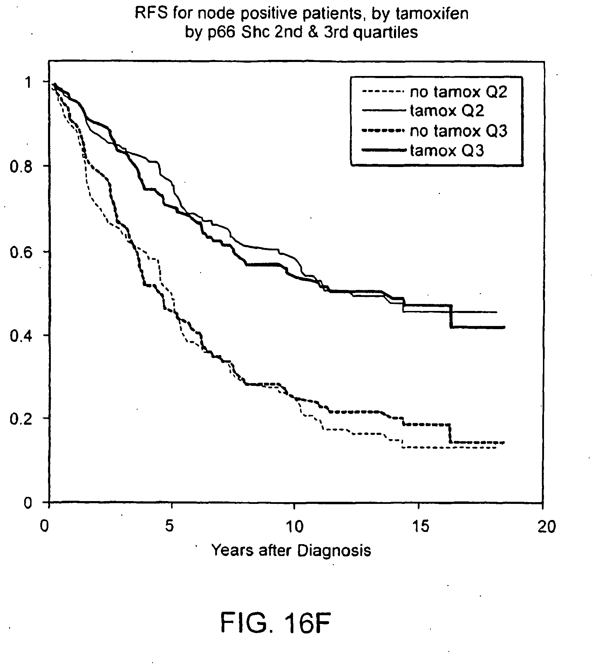

Results: In 1860 patients who did not receive adjuvant therapy, the Shc proteins were strong prognostic markers (hazard ratio (HR) = 2.6, 95% confidence intervals (CI) 1.1-6.0, P value (P) = 0.026) in multivariate models for RFS and DSS adjusted for nodal status, Her2 and estrogen receptor (ER) expression, patient age, tumor size and grade. Analysis of relapse-free survival (RFS) and disease specific survival (DSS) used multivariate models under assumptions of both log-normality as well as proportionality of hazard rate. The Shc proteins demonstrated very strong ability to predict patients' response to tamoxifen therapy in three separate cohorts of patients: 1) In the RWMC cohort, the Shc proteins provided an interquartile hazard ratio (HR) of 8.0 (95%CI 2.3-28, P=0.001); 2) In the MGH cohort, an interquartile HR of 9.1 (1.4-67 95% CI, P=0.02); 3) In the TMA study, the 12% of patients classified as high risk by the Shc Test were strongly protected by tamoxifen (0.26 HR, 0.096-0.71 95% CI, P = 0.009 in node positive patients; see Fig. 16), relative to the 63% patients in the low risk group who derived little benefit from tamoxifen.

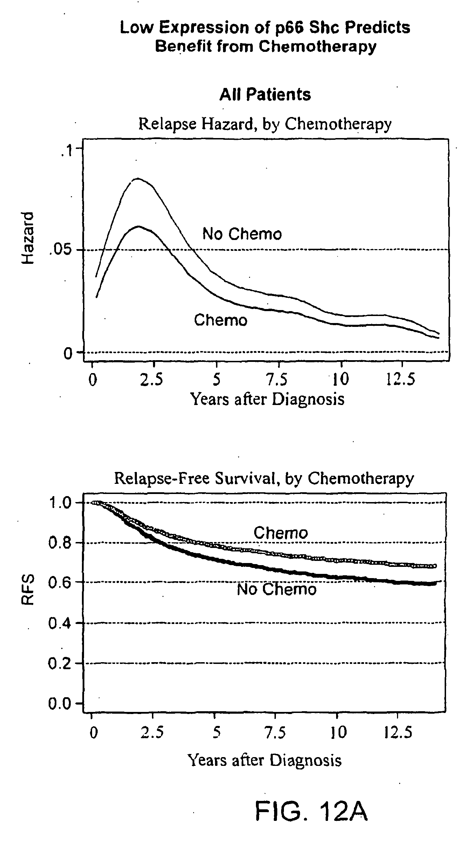

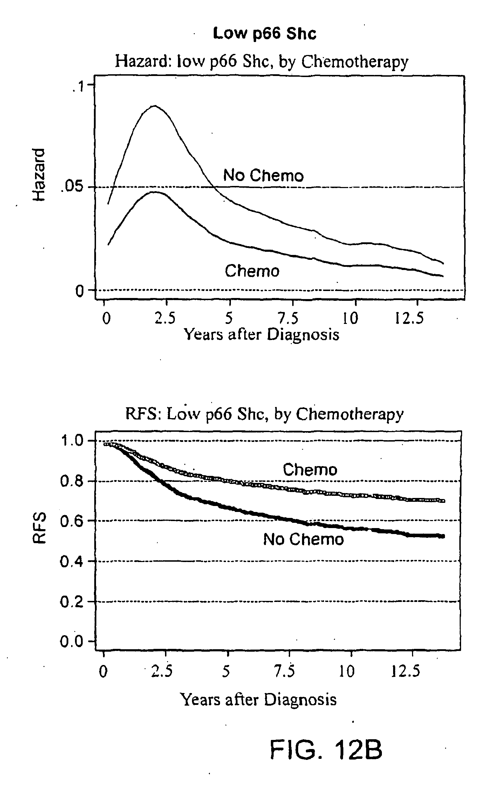

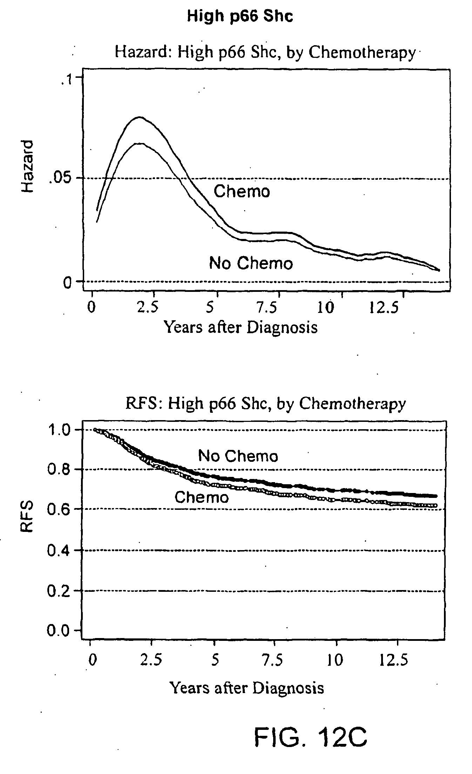

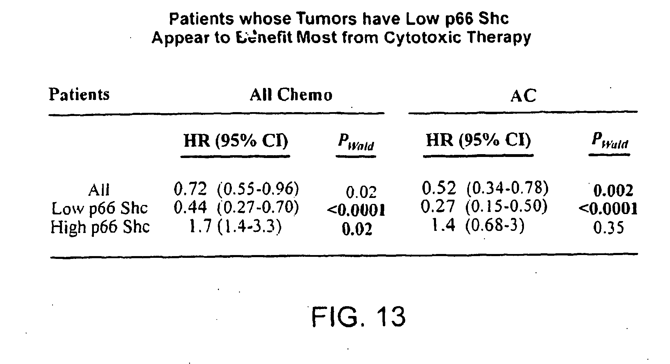

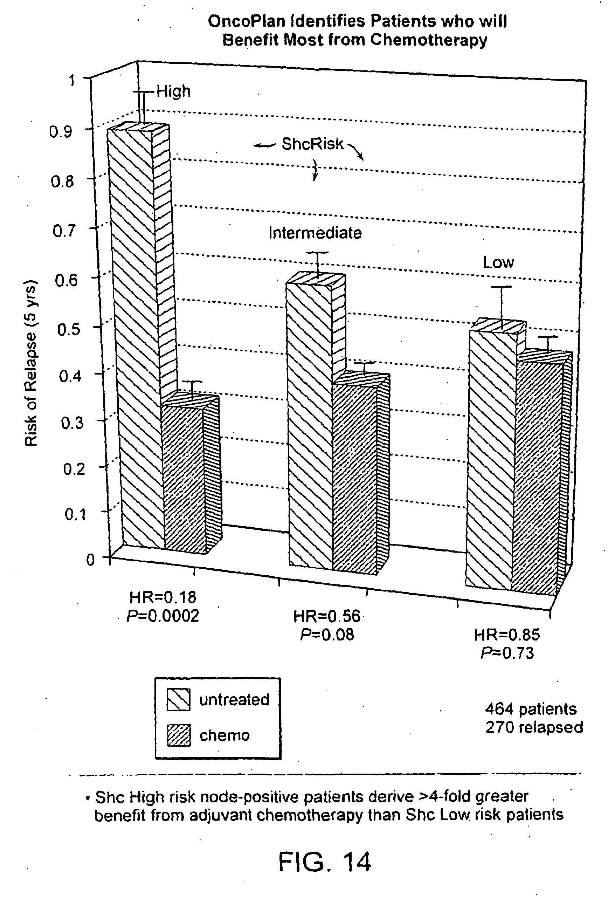

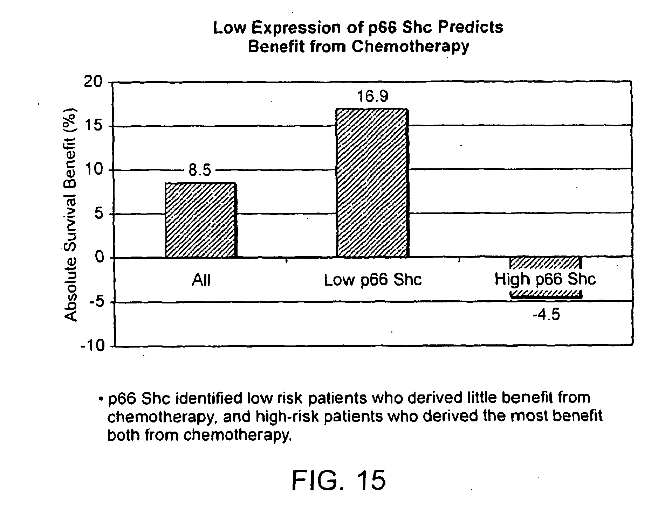

Similar analysis revealed that chemotherapy (either cytoxan, methotrexate, fluorouracil (CMF) or adriamycin cytoxan (AC)) was of little benefit to patients identified as low risk by the Shc Test, relative to patients identified as high risk received a 4-fold protective effect (0.25 HR, 0.08=0.78 95% CI, P=0.016). See Figs. 12-15.

Conclusions: Considered together, multiple studies on more than 3000 patients demonstrate that tumor levels of the Shc proteins identify: i) the large majority of patients who have a low risk profile and who will derive little benefit from adjuvant tamoxifen or chemotherapy; ii) the minority of patients who have a high risk profile, but who will derive the most benefit both from tamoxifen and cytotoxic adjuvant therapy.

### Example 2

Shc Proteins Predict Outcome in Treatment-Naïve Breast Cancer Patients and Chemotherapy Benefit

Better predictors of adjuvant chemotherapy would improve clinical management of breast cancer patients. The Shc proteins, implicated in many pathways associated with aggressive cancers, can be measured in tumors by the OncoPlan^{™} immunohistochemical test, which reports on phosphorylated (PY)-Shc and an inhibitory form of the protein, p66Shc. Previous studies have shown that OncoPlan^{™} is a strong predictor of disease outcome. Described herein is the ability of p66 Shc to identify those patients most likely to benefit from chemotherapy.

Methods: Population-based primary invasive tumors from 2380 women from British Columbia (BC) were arrayed in TMA format (no adjuvant therapy = 1663; chemotherapy (CMF, AC, FAC, CEF) = 717; relapsed = 854; died of disease = 501; 13 years median follow-up). Shc staining was scored on a continuous 0-5 scale by two pathologists, blinded to patient data.

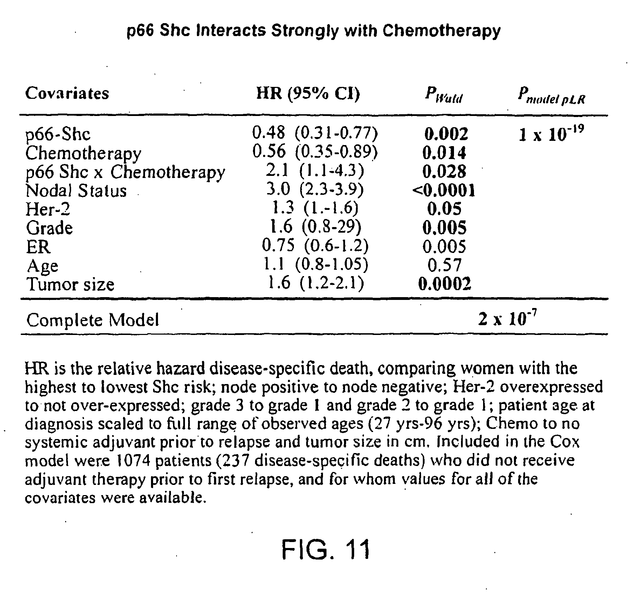

Results: In 1663 patients not receiving chemotherapy prior to first relapse, p66 Shc as a continuous variable had strong ability to predict relapse-free survival (RFS) (hazard ratio (HR) = 0.64, 95% CI 0.44-0.93, P = 0.018) and disease-specific survival (DSS) (HR = 0.47, 95% CI 0.30-0.75, P = 0.001) in multivariate Cox models adjusted for nodal status, Her2 and ER status, age, tumor size and grade. In contrast, p66 Shc did not predict outcome in chemotherapy-treated patients (RFS HR = 1.06, 95% CI 0.67 -1.65, P = 0.81; DSS HR = 1.00 95% CI 0.61-1.67, P = 0.98). This indicated an interaction between p66 Shc and chemotherapy, and indicated that p66 Shc levels predicted tumor responsiveness to chemotherapy. To test this hypothesis, a formal interaction analysis that demonstrated a significant interaction between p66 Shc and chemotherapy in which increasing values of p66 Shc decreased the ability of chemotherapy to improve patient outcome (see Fig. 11) was carried out. Formal demonstration of interaction then justified examining chemotherapy's protective ability in patients subgrouped by p66 Shc levels, using a fully adjusted Cox model.

The 60% of patients classified as high risk by OncoPlan^{™} (low p66 Shc levels) were strongly protected by chemotherapy (RFS HR = 0.53, 95% CI 0.38 - 0.75, P = 0.0001; DSS HR = 0.51, 95% CI 0.34 - 0.74, P = 0.001), whereas for the 40% of patients classified as low risk by OncoPlan^{™}, chemotherapy appeared to be a hazard (RFS HR = 1.2, 95% CI 0.79 - 1.8, P = 0.41; DSS HR = 1.9, 95% CI 1.15-3.02, P = 0.01). For the total population of patients, chemotherapy appeared to be protective (RFS HR = 0.74, 95% CI 0.59-94, P = 0.01; DSS HR = 0.81, 95% CI 0.62 - 1.05, P = 0.12). By drug, high risk patients were protected by AC (DSS HR = 0.31, 95% CI 0.017 - 0.57, P < 0.0001) and CMF (DSS HR = 0.48, 95% CI 0.30 - 0.76, P = 0.002); low risk patients were not protected by AC or CMF (HR > 1.5, P > 0.10). See Figs. 12-15.

Conclusions: These data indicate that p66 Shc levels in primary tumors identify patients with a low risk profile who will derive little benefit from chemotherapy and also patients with a high risk profile, who will derive the most benefit.

Example 3 Proposed molecular mechanisms whereby tumors displaying low p66 Shc are more sensitive to DNA-damaging therapies than tumors with high p66 Shc expression

### Summary:

While not wishing to be bound by theory, a tumor evolution model is described wherein oxidative stress and DNA damage provide a major selective pressure for tumors that have low levels of p66 Shc expression, and are aggressive if untreated, but respond well to systemic adjuvant therapy. The model nicely fits the data observations described herein for partitioning of naïve risk and chemotherapeutic benefit realized for patients whose tumors have either low or high levels of p66 Shc. The model makes several additional predictions concerning the distribution of markers for anti-apoptotic and pro-apoptotic factors relative to p66 Shc levels. These predictions were tested using data from the Vancouver General Hospital's 400-patient dataset.

### Model:

Normal cells that are under oxidative stress are programmed to self-destruct, by undergoing apoptosis. Such oxidative stress can be generated by chronic activation of growth factor pathways, by infiltrating neutrophils and macrophages, by neovascularization of hypoxic tumors, and by re-perfusion of ischemic tissues (Brown, N.S., et al., Breast Cancer Res., 3:323-327 (2001); Irani, K., et al., Science, 275:1649-1652 (1997)).

Both p66 Shc protein (and its phosphorylation on serine36) and TP53 are required for the normal apoptotic response of cells under oxidative stress (Purdom, S., et al., Trends Mol. Med, 9:206-210(2003); Migliaccio, E., et al, Nature:402:309-313 (1999); Nemoto, S., et al., Science, 295:2450-2452 (2002); Pellegrini, M., et al., Apoptosis, 10:13-18 (2005)). *In vitro* model systems indicate that in the absence of both p66 Shc and TP53, cells evidence no apoptotic response to oxidative stress (Trinei, M., et al., Oncogene, 21:3872-3878 (2002)). The presence of either p66 Shc or TP53 alone provide a significant but muted response, while the presence of both demonstrate a synergistic response. This synergism may be explained by the following observations:

### 1. p66 Shc phosphorylation on serine36:

a. up-regulates HIF-1; HIF-1 stabilizes TP53, increasing its steady-state levels in the cell;
b. up-regulates other pro-apoptotic factors, including Bax, GADD45 and FasL (Pacini, S., et al., Mol. Cell Biol., 24:1747-1757 (2004));
c. down-regulates anti-apoptotic factors, including Bcl;
d. inhibits the ability of the FKHRL1 transcription factor to upregulate cellular levels of ROS-scavenger pathways that would reduce oxidative stress (e.g. catalase) (Nemoto, S., et al., Science, 295:2450-2452 (2002));
e. translocates p66 Shc to the mitochondria where, in combination with HRP70, it shunts electrons into the cell cytoplasm, increasing stress; it causes release of cytochrome c from the mitochondria (Trinei, M., et al., Oncogene, 21:3872-3878 (2002)).

### 2. TP53 expression

a. up-regulates p66 Shc expression by increasing p66 Shc stability (Trinei, M., et al., Oncogene, 21:3872-3878 (2002));
b. up-regulates several other pro-apoptotic factors;
c. down-regulates anti-apoptotic factors.

The evidence discussed above indicates that aggressive tumors that are continually experiencing such oxidative stress would have a selective advantage if they displayed low levels of p66 Shc, and thereby avoided apoptotic death. Consistent with this notion, low levels of p66 Shc have been found in breast cancer cell lines that possessed over-expressed, activated Her2/neu, or possessed autocrine growth factor loops (Stevenson, L.A., et al., Breast Cancer Research & Treatment, 49:119-128 (1998)), and in aggressive breast cancer (Davol, P.A., et al., Cancer Res., 63:6772-6783 (2003); Frackelton, A.R., et al., San Antonio Breast Cancer Symposium:1124a (2005); Frackelton, A.R., Proc. Amer. Assoc. Cancer Res., 46:LB201 (2005)).

Alternatively, aggressive tumors under severe oxidative stress could maintain p66 Shc expression at high levels and avoid apoptosis if its tumor cells utilized other mechanisms to resist apoptosis. Thus, tumors with high p66 Shc might avoid or minimize oxidative-stress-induced apoptosis if they contained dis-regulated or mutationally inactivated TP53, down-regulated pro-apoptotic factors or up-regulated anti-apoptotic factors such as Bcl-2.

Thus, in the absence of systemic adjuvant therapy, it is expected that tumors with low levels of p66 Shc would be vital and aggressive. However, these same tumors would be relatively unlikely to have independently been selected for reduced expression of pro-apoptotic factors (e.g. Bax or TP53), or increased expression of anti-apoptotic factors.(e.g. Bcl-2) since it would not have been necessary for survival from oxidative stress if p66 Shc were already down-regulated. Thus, because apoptosis in response to DNA-damaging agents generally requires TP53, but does not require p66 Shc, these same cells with low p66 Shc but otherwise normal functioning apoptotic pathways would be expected to be very sensitive to DNA-damaging therapeutics.

Conversely, tumors with high p66 Shc should be separable into two general classes. Class I high p66 Shc tumors would tend to be non-aggressive, have normal apoptotic pathways largely intact, and would be under little oxidative stress. These patients would tend to be cured by their surgical (and radio-) therapy, and thus show no additional benefit from DNA-damaging adjuvant therapeutics. Class II high p66 Shc tumors would tend to be aggressive tumors under high oxidative stress that avoid apoptotic death by down-regulating pro-apoptotic pathways (e.g. p53), or upregulating anti-apoptotic pathways (e.g. Bcl-2), or both. Patients whose tumors were Class II high p66 Shc would be expected to have poor pure prognoses after surgical (and radio-) therapies, and to receive relatively little benefit from DNA-damaging adjuvant therapeutics.

Thus, to the extent that the majority of patients with high p66 Shc are Class I and a minority are Class II, patients with high p66 Shc would have a favorable pure prognosis, but benefit little from DNA-damaging therapies.

This model, then, would appear to nicely explain the observations described herein (see Figs. 12-15).

In addition to their effects on apoptosis, p66 Shc and TP53 affect cell proliferation. This is well established for TP53, which senses DNA damage and pauses the cell cycle to await repair if damage is not too extensive. TP53-mutated tumors proliferate more rapidly than TP53 normal cells, which contributes to their aggressiveness. Normally, rapidly proliferating cells are more sensitive to DNA-damaging therapeutics that typically exert their effects only during S-phase of the cell cycle. However, TP53 mutant cells are relatively insensitive to this. The effect of the p66 Shc protein on cell proliferation is not so clear, but its ability to inhibit signaling through Shc-Grb2 complexes to Ras and to c-fos suggest that under certain circumstances it too may slow cell proliferation. To the extent that this is true, loss of p66 Shc might increase cell proliferation and thus increase the tumor cell's vulnerability to DNA-damaging therapeutics.

## Claims

1. An *in vitro* method for predicting whether an individual's chances of relapsing or dying from breast cancer are reduced two fold or more, compared to the general population of cancer patients having the same cancer or a substantially similar cancer, if the individual receives a treatment for breast cancer wherein the treatment comprises administering one or more agents to the individual, comprising:
determining the amount of p66-Shc present in cancerous cells of the individual,
wherein if the amount of p66-Shc present in the cancerous cells of the individual is lower than the amount of p66-Shc in a control sample taken from one or more individuals that do not have breast cancer,
then the individual's chances of relapsing or dying from breast cancer are reduced if the individual receives the treatment.
.

2. The method of Claim 1, wherein the treatment is a hormonal treatment.

3. The method of Claim 1, wherein the treatment is a chemotherapeutic treatment.

4. The method of Claim 2, wherein the hormonal treatment comprises tamoxifen.

5. The method of Claim 43, wherein the chemotherapeutic treatment comprises adriamycin cytoxan (A).

6. The method of Claim 43, wherein the chemotherapeutic treatment comprises an agent selected from the groups consisting of: cytoxan (C), methotrexate (M), fluorouracil (F), epirubicin (E) and a combination thereof.

7. The method of Claim 1, wherein the amount of p66-Shc present in cancerous cells of the individual is determined using immunohistochemistry.

8. The method of Claim 7, wherein the immunohistochemistry is performed using an antibody or antigen binding fragment thereof, that specifically binds to the p66-Shc.

9. The method of Claim 8, wherein the antibody is a monoclonal antibody.

10. The method of Claim 8, wherein the antibody comprises a label.

11. The method of Claim 1, wherein the individual is human.

12. The method of Claim 1, wherein the treatment for cancer is an adjuvant therapy.

## Patentansprüche

1. *In vitro*-Verfahren zur Vorhersage, ob die Chancen eines Individuums, einen Rückfall zu erleiden oder an Brustkrebs zu sterben zweifach oder mehr reduziert sind im Vergleich zur allgemeinen Population von Krebspatienten, die den selben Krebs oder einen im Wesentlichen ähnlichen Krebs haben, wenn das Individuum eine Brustkrebsbehandlung erhält, wobei die Behandlung die Verabreichung eines oder mehrerer Agenzien an das Individuum umfasst, wobei das Verfahren umfasst:
Bestimmen der Menge an p66-Shc, das in Krebszellen des Individuums vorliegt, wobei, wenn die Menge an p66-Shc, die in den Krebszellen des Individuums vorliegt, geringer ist als die Menge an p66-Shc in einer Kontrollprobe, die von einem oder mehreren Individuen entnommen wurde, die keinen Brustkrebs haben,
dann die Chancen des Individuums, einen Rückfall zu erleiden oder an Brustkrebs zu sterben, reduziert sind, wenn das Individuum die Behandlung erhält.

2. Verfahren nach Anspruch 1, wobei die Behandlung eine hormonelle Behandlung ist.

3. Verfahren nach Anspruch 1, wobei die Behandlung eine chemotherapeutische Behandlung ist.

4. Verfahren nach Anspruch 2, wobei die hormonelle Behandlung Tamoxifen umfasst.

5. Verfahren nach Anspruch 3, wobei die chemotherapeutische Behandlung Adriamycin-Cytoxan (A) umfasst.

6. Verfahren nach Anspruch 3, wobei die chemotherapeutische Behandlung ein Agens umfasst, das aus der Gruppe ausgewählt ist, die besteht aus: Cytoxan (C), Methotrexat (M), Fluoruracil (F), Epirubicin (E) und eine Kombination davon.

7. Verfahren nach Anspruch 1, wobei die Menge an p66-Shc, die in den Krebszellen des Individuums vorliegt, unter Verwendung von Immunhistochemie bestimmt wird.

8. Verfahren nach Anspruch 7, wobei die Immunhistochemie unter Verwendung eines Antikörpers oder Antigenbindefragments davon durchgeführt wird, das spezifisch an p66-Shc bindet.

9. Verfahren nach Anspruch 8, wobei der Antikörper ein monoclonaler Antikörper ist.

10. Verfahren nach Anspruch 8, wobei der Antikörper eine Markierung umfasst.

11. Verfahren nach Anspruch 1, wobei das Individuum ein Mensch ist.

12. Verfahren nach Anspruch 1, wobei die Behandlung des Krebs eine adjuvante Therapie ist.

## Revendications

1. Méthode *in vitro* pour prévoir si la probabilité d'un individu de présenter une récidive, ou de mourir, du cancer du sein est réduite d'un facteur égal ou supérieur à deux, comparativement à la population générale de patientes atteintes d'un cancer, présentant le même cancer ou un cancer substantiellement similaire, si l'individu reçoit un traitement pour le cancer du sein, comprenant :
la détermination de la quantité de p66-Shc présente dans des cellules cancéreuses de l'individu,
où, si la quantité de p66-Shc présente dans les cellules cancéreuses de l'individu est inférieure à la quantité de p66-Shc dans un échantillon-témoin prélevé chez un ou plusieurs individus n'ayant pas de cancer du sein,
alors la probabilité de l'individu de présenter une récidive, ou de mourir, du cancer du sein est réduite si l'individu reçoit le traitement.

2. Méthode suivant la revendication 1, dans laquelle le traitement est un traitement hormonal.

3. Méthode suivant la revendication 1, dans laquelle le traitement est un traitement chimiothérapeutique.

4. Méthode suivant la revendication 2, dans laquelle le traitement hormonal comprend l'utilisation de tamoxifène.

5. Méthode suivant la revendication 3, dans laquelle le traitement chimiothérapeutique comprend l'utilisation d'adriamycine-cytoxan (A).

6. Méthode suivant la revendication 3, dans laquelle le traitement chimiothérapeutique comprend un agent choisi dans le groupe consistant en le cytoxan (C), le méthotrexate (M), le fluorouracile (F), l'épirubicine (E) et une de leurs associations.

7. Méthode suivant la revendication 1, dans laquelle la quantité de p66-Shc présente dans des cellules cancéreuses de l'individu est déterminée en utilisant l'immunohistochimie.

8. Méthode suivant la revendication 7, dans laquelle l'immunohistochimie est effectuée en utilisant un anticorps ou son fragment de liaison à l'antigène, qui se lie spécifiquement au p66-Shc.

9. Méthode suivant la revendication 8, dans laquelle l'anticorps est un anticorps monoclonal.

10. Méthode suivant la revendication 8, dans laquelle l'anticorps comprend un marqueur.

11. Méthode suivant la revendication 1, dans laquelle l'individu est un être humain.

12. Méthode suivant la revendication 1, dans laquelle le traitement du cancer est un traitement adjuvant.
